# EUROPEAN PATENT APPLICATION

(11) **EP 2 298 777 A2**
(43) Date of publication of application: **23.03.2011**
(21) Application number: 09762643.6
(22) Date of filing: 09.06.2009
(51) Int. Cl.: C07D 495/04

(54) **METHOD FOR PREPARING CLOPIDOGREL AND ITS DERIVATIVES**

(30) Priority: 09.06.2008 KR 20080053815
(71) Applicant: Enzytech, Ltd., Daejeon 306-230 (KR)
(72) Inventor: HWANG, Soon Ook, Daejeon 305-761 (KR); KIM, Young Jin, Daejeon 302-821 (KR)
(74) Representative: Behnisch, Werner
(86) International application number: PCT/KR2009/003083
(87) International publication number: WO 2009/151256

(57) **Abstract**

The present invention relates to a method for preparing Clopidogrel and its derivatives. More particularly, the present invention is a method for preparation of (S)-2-Clopidogrel and its derivatives, which are active inhibitors of platelet aggregation, from an optically active (S)-2-chlorophenyl glycine alkyl ester through hydrolysis of racemic 2-chlorophenylglycine alkyl esters using an enzyme. The present invention employs a simple procedure to prepare Clopidogrel and its derivatives. Because no chiral resolving agents are used except for a small amount of enzyme, the cost of preparation can be reduced. In addition, the present invention is suitable for synthesizing highly optical-active Clopidogrel and its derivatives on a large scale by using optically active (S)-2-chlorophenylglycine alkyl ester obtained in high yield as an intermediate, and is also environmentally friendly since no highly toxic reagents are employed.

## Description

### [Technical Field]

The present invention relates to a method for preparing Clopidogrel and derivatives thereof. More particularly, the present invention relates to a method for preparing (S)-Clopidogrel as an inhibitor of platelet aggregation and derivatives thereof, the method including subjecting racemic 2-chlorophenylglycine alkyl ester to enzymatic hydrolysis to form optically active (S)-2-chlorophenylglycine alkyl ester as an intermediate, and providing (S)-Clopidogrel and derivatives thereof from the intermediate.

### [Background Art]

Clopidogrel is represented by the following Chemical Formula 1, wherein R₁ is methyl group, and has a chemical name of methyl-(S)-α-(o-chlorophenyl)-6,7-dihydrothieno[3,2-c] pyridine-5(4H)-acetate. In addition, Clopidogrel shows strong platelet aggregation inhibitory activity and anti-thrombotic activity, and thus it has been used as an agent for treating vascular system diseases, including peripheral arterial diseases, such as stroke, thrombosis and embolism, and coronary arterial diseases, such as myocardial infarction and angina.

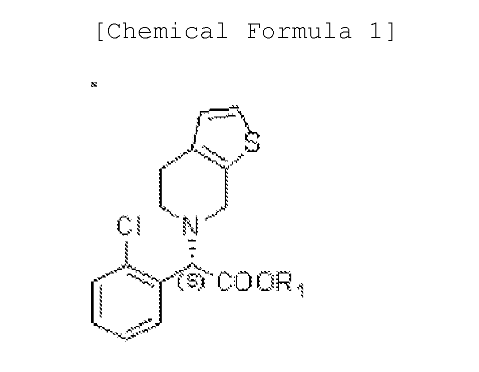

Clopidogrel is obtained in the form of racemate by providing methyl-2-(2-chlorophenyl)acetate in its 2-chloro or 2-bromo form, and by reacting 4,5,6,7-tetrahydrothieno[3,2-c]pyridine therewith (see, US Patent No. 4, 529, 596, US Patent No. 5, 036, 156 and US Patent No. 5, 189, 170) . However, Clopidogrel is effective as a treating agent only when it is present as its dextrorotatory optically active form, (S)-enantiomer. Accordingly, many attempts have been made to provide Clopidogrel as a pure optical isomer substantially free from its levorotatory isomer, (R)-enantiomer.

Among such attempts, US Patent No. 4,847,265 discloses a method for preparing (S)-Clopidogrel, which includes preparing racemic Clopidogrel, selectively forming a diastereomeric salt with camphorsulfonic acid as an optical isolating agent only from (S)-Clopidogrel, carrying out recrystallization to obtain a salt free from (R)-enantiomer, and removing the optical isolating agent with a weak base, such as NaHCO₃.

US Patent No. 5,204,469 discloses a method for preparing (S)-Clopidogrel, which includes: forming a diastereomeric salt of 2-chlorophenyl glycine or 2-chlorophenylclycine methyl ester with camphorsulfonic acid or tartaric acid as an optical isolating agent, followed by recrystallization; reacting the resultant diastereomeric salt with 2-thienylethyl p-toluenesulfonate to provide a compound represented by the following Chemical Formula 2; and carrying out cyclization of the compound of Chemical Formula 2 with formaldehyde to obtain (S)-Clopidogrel. In a variant, the method includes: providing methyl-2-(2-chloro phenyl)acetate in the form of its 2-chloro or 2-bromo form in the same manner as generally known in the art; reacting the resultant product with 2-thienylethyl p-toluenesulfonate to obtain a compound represented by the following Chemical Formula 2 in the form of racemate; forming a diastereomeric salt therefrom with camphorsulfonic acid or tartaric acid, followed by recrystallization; removing the optical isolating agent to obtain (S)-enantiomer (2); and carrying out cyclization of the (S)-enantiomer with formaldehyde in the same manner as described above to provide (S)-Clopidogrel.

US Patent No. 6,080,875 discloses a method for preparing a compound represented by the following Chemical Formula 2, which includes carrying out optical isolation of 2-chlorophenyl glycine methyl ester with 2,4-dinitrobenzoyl phenylglycine; removing the optical isolating agent with aqueous NaHCO₃ solution; and reacting the resultant product with sodium 2-thienyl glycidate to provide a compound of Chemical Formula 2.

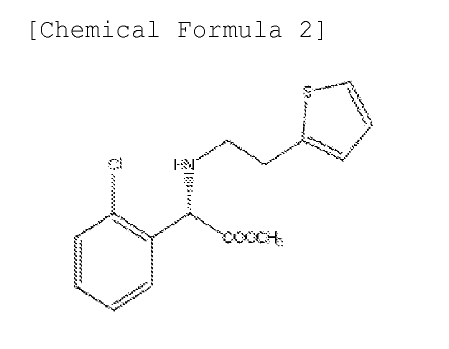

Similarly, WO 98/51689 discloses a method for preparing (S)-Clopidogrel, which includes: reacting 2-chlorobenzaldehyde with sodium cyanide; treating the resultant product with 2-thienylethylamine to provide an acetonitrile compound represented by the following Chemical Formula 3; carrying out optical isolation of the acetonitrile compound with camphorsulfonic acid, or converting the acetonitrile compound into an acetamide compound represented by the following Chemical Formula 4, followed by optical isolation thereof with tartaric acid; converting the isolated compound into a methyl ester compound represented by the above Chemical Formula 2; and carrying out cyclization with formaldehyde to provide (S)-Clopidogrel.

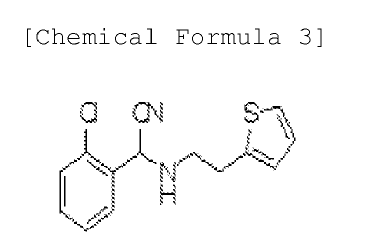

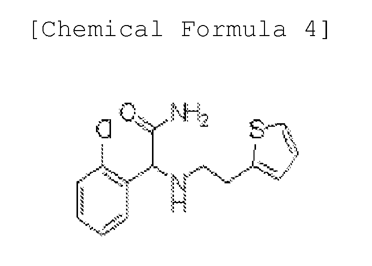

Meanwhile, Korean Unexamined Patent Publication No. 2007-0068043 discloses a method for preparing (S)-Clopidogrel, which includes: preparing Clopidogrel racemic carboxylate represented by the following Chemical Formula 5; converting the resultant compound into a diastereomeric salt by using cinchonine; extracting the diastereomeric salt with a suitable solvent under an acidic condition to perform optical isolation of (S)-Clopidogrel carboxylate; and treating the resultant product with methanol to provide (S)-Clopidogrel. In addition, Korean Unexamined Patent Publication No. 2006-0134541 discloses a method for preparing (S)-Clopidogrel, which includes: carrying out optical isolation of Clopidogrel racemic carboxylate represented by the following Chemical Formula 5 by using an optically active amine derivative, such as an expensive optical isolating agent, (1R,2R)-(-)-2-amino -1-phenyl-1,3-propane diol, to obtain (S)-Clopidogrel carboxylate; and treating the resultant product with methanol to obtain (S)-Clopidogrel.

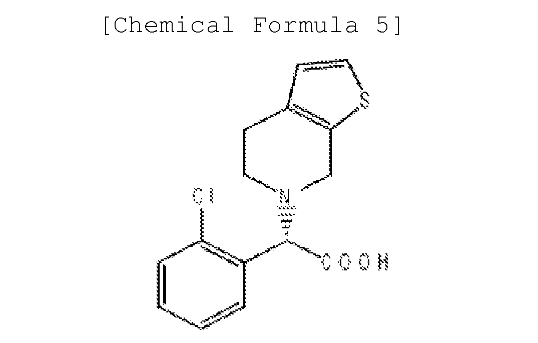

However, all of the above-mentioned methods are problematic in that they are based on optical isolation of racemates corresponding to Clopidogrel or intermediates thereof using an expensive optical isolating agent, and require a relatively long time up to several days to perform the optical isolation. In addition, according to such methods, the diastereomeric salt obtained at the initial time of the optical isolation shows insufficient optical purity, and thus should be further purified to obtain a sufficient pharmaceutically acceptable optical purity, resulting in a drop in yield. Moreover, camphorsulfonic acid used for the optical isolation is not recovered from the reaction mixture but is discarded, since it has high water solubility. In addition, cinchonine is highly toxic, and thus is not eco-friendly. Further, the above-mentioned methods use expensive reagents, such as 2-thienylethylamine, 2-thienylethyl alcohol or 2-thienylethyl bromide, to obtain desired (S)-enantiomers, while the corresponding (R)-enantiomers should be discarded. Ultimately, all of the above-mentionedmethods are not preferred in terms of cost-efficiency and eco-friendly characteristics.

Similarly, Korean Unexamined Patent Publication No. 2006-0098009 discloses a method for preparing (S)-Clopidogrel, which includes: reacting (S)-2-chlorophenylglycine methyl ester, which has been subjected to optical isolation using tartaric acid, with 2-thienyl acetate to obtain (S)-methyl--(2-thienylacetamide)-2-(2-chlorophenyl)acetate represented by the following Chemical Formula 6, reducing the amide functional group to obtain a compound represented by Chemical Formula 2, and carrying out cyclization by using formaldehyde to obtain (S)-Clopidogrel. However, this method still has the above-mentioned problems related to the optical isolation. Moreover, when reducing the amide functional group, methyl ester is also reduced, resulting in a rapid drop in yield. 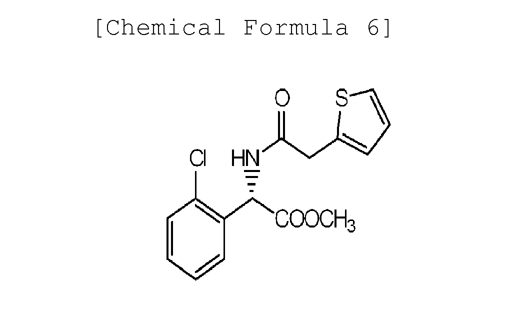 To overcome the above-mentioned problems, in the method for preparing (S)-Clopidogrel disclosed in WO99/18110, (R)-methyl-2-chloromandelate sulfone derivative is allowed to react with 2-thienylethylamine to form an intermediate of the above Chemical Formula 2, followed by cyclization using formaldehyde. Otherwise, 4,5,6,7-tetrahydrothieno[3,2-c] pyridine is used to obtain (S)-Clopidogrel represented by Chemical Formula 1. Additionally, in the method disclosed in WO 03/093276, N , N'-bis-4,5,6,7-tetrahydrothieno[3,2-c] pyridylmethane is allowed to react with (R)-methyl-2-bromo -2-(2-chlorophenyl)acetate or (R)-2-chloro phenylacetate sulfone derivative to obtain (S)-Clopidogrel. However, these methods are not amenable to mass production, since they may form racemate depending on the leaving group, i.e., sulfonic acid derivative or halogen, and the reaction condition, resulting in a drop in optical purity.

In addition, US Patent No. 6,858,734 discloses a method for preparing (S)-chlopidogrel, which includes: reacting 2-chlorobenzaldehyde with 2-thienylethylamine to obtain a compound represented by the following Chemical Formula 7; carrying out asymmetric synthesis by using Strecker catalyst to form a (S) -enantiomeric compound represented by the following Chemical Formula 8; carrying out cyclization with formaldehyde; and converting the nitrile compound into methyl ester to obtain (S)-Clopidogrel. However, the above method is problematic in that it requires use of highly toxic cyanic acid and provides an optical purity of merely about 85%.

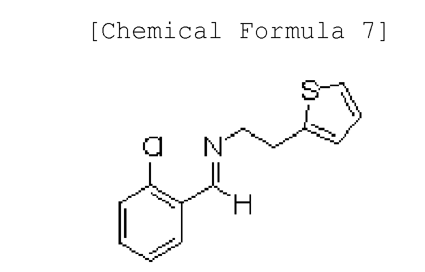

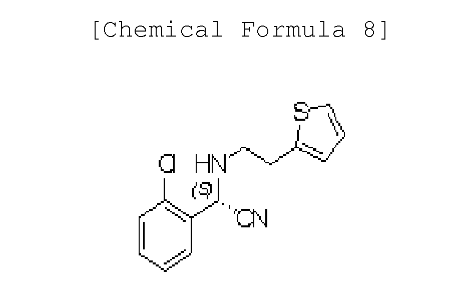

Under these circumstances, the present inventors have conducted intensive studies to overcome the above-mentioned problems. We have found that it is possible to obtain (S)-Clopidogrel as an inhibitor of platelet aggregation and derivatives thereof with ease by subjecting racemic 2-chlorophenylglycine alkyl ester to enzymatic hydrolysis in an aqueous solution or in an aqueous solution containing a solvent to form optically active (S)-2-chlorophenylglycine alkyl ester compound, and by providing (S)-Clopidogrel and derivatives thereof from the optically active compound as an intermediate. The present invention is based on this finding.

### [Disclosure]

### [Technical Problem]

The present invention has been made to solve the problems occurring in the related art, and an object of the present invention is to provide a method for preparing (S)-Clopidogrel and derivatives thereof having high optical purity by a simple process with low cost.

### [Technical Solution]

In order to accomplish the above objects, according to one embodiment of the present invention, there is provided a method for preparing (S)-Clopidogrel represented by the following Chemical Formula 1, or derivatives or salts thereof, the method including: (a) subjecting a racemic 2-chlorophenylglycine alkyl ester compound represented by Chemical Formula 9 to enzymatic hydrolysis to obtain an optically active compound represented by the following Chemical Formula 10; (b) reacting the optically active compound represented by Chemical Formula 10 with a compound represented by the following Chemical Formula 11 to obtain a compound represented by the following Chemical Formula 12; and (c) carrying out cyclization of the compound represented by Chemical Formula 12 with a formylating agent in the presence of an acid.

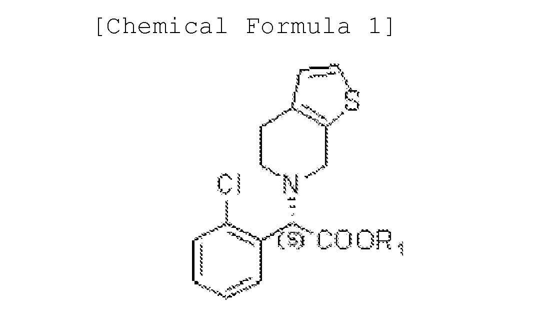

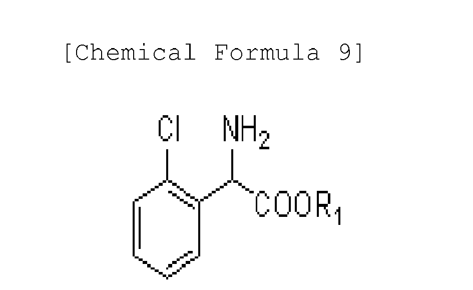

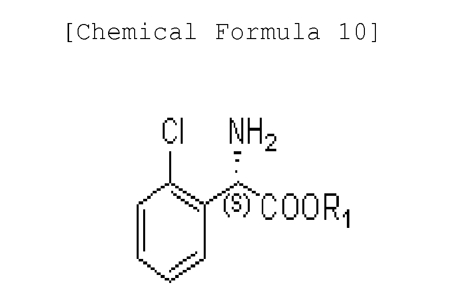

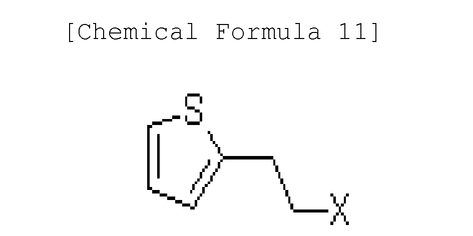

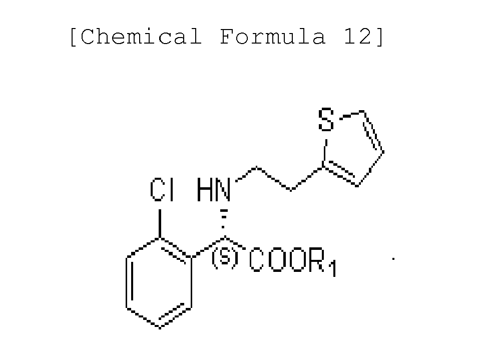

In the above Chemical Formulae, R₁ is H, substituted or non-substituted C1-C8 alkyl, substituted or non-substituted C1-C8 alkenyl, benzyl or C3-C6 cycloalkyl; and X is a halogen atom selected from the group consisting of fluorine (F), chlorine (Cl), bromine (Br) and iodine (I) or -OSO₂R₂ (wherein R₂ is substituted or non-substituted C1-C8 alkyl, substituted or non-substituted aryl, substituted or non-substituted arylalkyl, substituted or non-substituted heteroaryl or substituted or non-substituted heteroarylalkyl).

### [Best Mode]

### [Mode for Invention]

Hereinafter, exemplary embodiments of the present invention will be described in detail.

The present invention provides a method for preparing (S)-Clopidogrel represented by the following Chemical Formula 1, or derivatives or salts thereof, the method including: (a) subjecting a racemic 2-chlorophenylglycine alkyl ester compound represented by Chemical Formula 9 to enzymatic hydrolysis to obtain an optically active compound represented by the following Chemical Formula 10; (b) reacting the optically active compound represented by Chemical Formula 10 with a compound represented by the following Chemical Formula 11 to obtain a compound represented by the following Chemical Formula 12; and (c) carrying out cyclization of the compound represented by Chemical Formula 12 with a formylating agent in the presence of an acid.

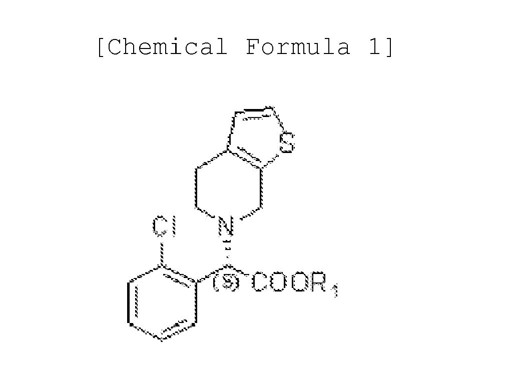

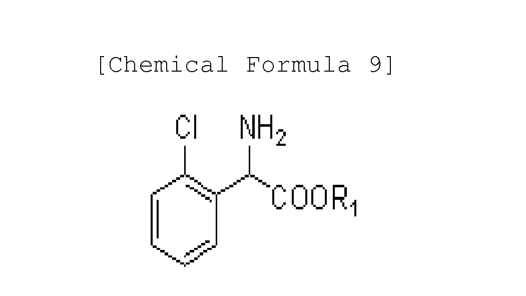

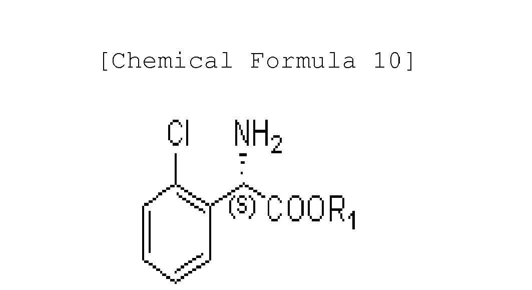

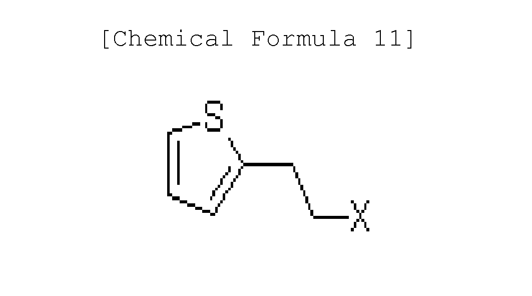

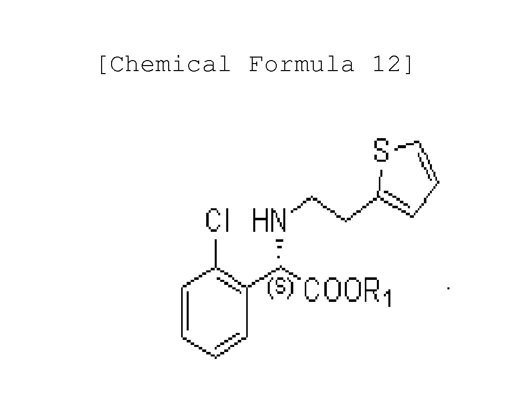

In the above Chemical Formulae, R₁ is H, substituted or non-substituted C1-C8 alkyl, substituted or non-substituted C1-C8 alkenyl, benzyl or C3-C6 cycloalkyl; and X is a halogen atom selected from the group consisting of fluorine (F), chlorine (Cl), bromine (Br) and iodine (I) or -OSO₂R₂ (wherein R₂ is substituted or non-substituted C1-C8 alkyl, substituted or non-substituted aryl, substituted or non-substitutedarylalkyl, substituted or non-substituted heteroaryl or substituted or non-substituted heteroarylalkyl).

According to an embodiment of the present invention, Clopidogrel, or derivatives or salts thereof may be obtained by the method as depicted in the following Reaction Scheme 1:

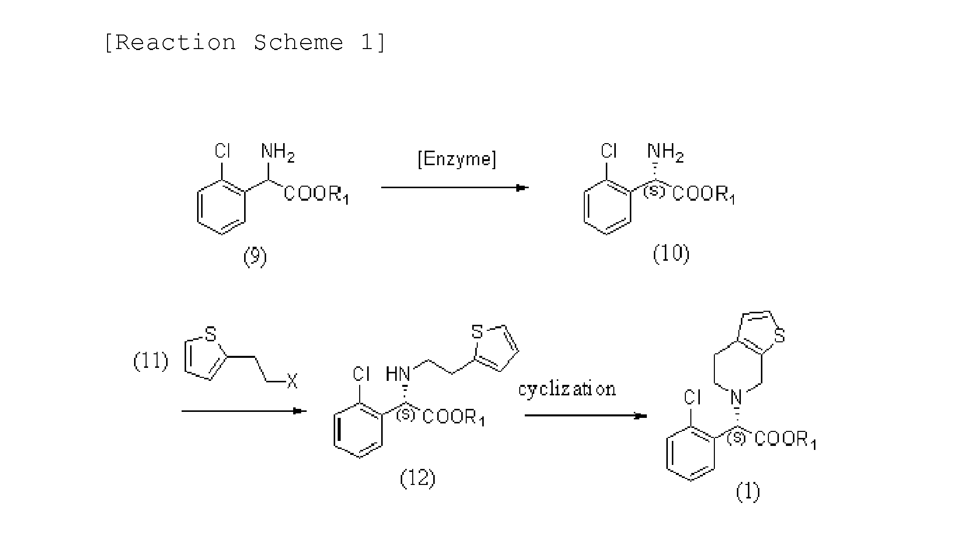

In the above Reaction Scheme 1, R₁ and X have the same meanings as defined above.

As shown in Reaction Scheme 1, racemic 2-chlorophenylglycine alkyl ester compound represented by Chemical Formula 9 is subjected to hydrolysis using an enzyme effective for hydrolysis in aqueous solution or an aqueous phase containing a solvent to provide optically active (S)-chlorophenylglycine alkyl ester compound (Compound 10) as an intermediate; the resultant (S)-2-chlorophenylglycine alkyl ester compound (Compound 10) is allowed to react with a compound represented by Chemical Formula 11 to provide a compound represented by Chemical Formula 12; and subjecting the compound of Chemical Formula 12 to cyclization to obtain (S) -Clopidogrel as an inhibitor of platelet aggregation, or salts or derivatives thereof (Chemical Formula 1).

More particularly, (S)-Clopidogrel represented by Chemical Formula 1, or derivatives of salts thereof according to the present invention can be obtained by using 2-chlorophenylglycine alkyl ester compound represented by Chemical Formula 9, which is commercially available or prepared easily with low cost, as a starting material. The 2-chlorophenylglycine alkyl ester compound is subjected to stereoselective hydrolysis in an aqueous solution or an aqueous phase containing a solvent in the presence of a hydrolase or strain containing the same to provide optically active (S)-2-chlorophenylglycine alkyl ester or (S)-2-chlorophenyl glycine represented by Chemical Formula 10.

According to an embodiment, the hydrolysis is carried out under pH of 4-10 at a temperature of 10-70°C. When the hydrolysis is carried out under pH and temperature conditions away from the above ranges, unstabilization of enzymes used for the hydrolysis or side-reactions may occur.

According to a particular embodiment, the enzyme includes but is not limited to: a hydrolase, such as *protease* or *lipase.* Particular examples of the hydrolase include *flavourzyme, protease A, alcalase, savinase, protamex, esperase, novozyme* 435 (available from Novozyme), esterase, *acylase* and combinations thereof. Microorganisms containing such hydrolases may also be used as the enzyme source. Particular examples of such microorganism strains include *Bacillus* sp, *Aspergillus* oryzae, *Aspergillus niger, Candida antarctica* and combinations thereof. However, any strains or microorganisms may be used with noparticular limitation, as long as they contain hydrolase.

After carrying out the stereoselective hydrolysis according to the present invention, (S)-2-chlorophenylglycine alkyl ester and (S)-2-chlorophenylglycine are extracted with high optical purity by using a solvent selected from the group consisting of ethyl acetate, methylene chloride, diethyl ether, diisopropyl ether, tert-butyl methyl ether, 1,2-dimethoxyethane, 1,2-dichloroethane, benzene, toluene, xylene and mixtures thereof so that they may be separated with ease from (R) -2-chlorophenylglycine alkyl ester and (R)-2-chlorophenylglycine.

The resultant (S)-2-chlorophenylglycine alkyl ester and (S)-2-chlorophenylglycine with high optical purity is allowed to react with a compound represented by Chemical Formula 11 in Reaction Scheme 1, such as 2-thienylethyl sulfone derivative or 2-thienylethyl halogen derivative to provide a compound represented by Chemical Formula 12. Then, the compound of Chemical Formula 12 is subjected to cyclization using a formulating agent in the presence of acid according to the method known to those skilled in the art to provide a target product, (S)-Clopidogrel derivative, represented by Chemical Formula 1.

According to an embodiment, the formylating agent may be selected from the group consisting of formaldehyde, formaldehyde hydrate and formaldehyde polymers. The acid is used for preparing a salt, and particular examples of the acid include inorganic acid or organic acid, such as sulfuric acid, hydrochloric acid, hydrobromic acid, sulfonic acid, formic acid, acetic acid, or the like.

As used herein, salts of (S)-Clopidogrel means the conventional acid addition salts of Clopidogrel as described in EP 0,281,459 or International Patent Publication No. W02004/074215, regardless of the solidity or stability of the salts. Typical examples of the inorganic acid used for forming such salts include hydrochloric acid, hydrobromic acid, hydroiodic acid, nitric acid, sulfuric acid, phosphoric acid, hypophosphoric acid, or the like. In addition, various salts derived from organic acids, such as aliphatic mono- and di-carboxylic acid, phenyl-substituted alkanoic acid, hydroxyalkanoic acid and hydroxyalkanedioic acid, aromatic acids, aliphatic and aromatic sulfonic acid, may also be used. Therefore, such pharmaceutically acceptable salts may include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, beta-hydroxybutyrate, chloride, cinnamate, citrate, formate, fumarate, glycolate, heptanoate, lactate, maleate, hydroxymaleate, malonate, mesylate, nitrate, oxalate, phthalate, phosphate, monohydrogenphosphate, dihydrogenphosphate, metaphosphate, pyrophosphate, propionate, phenylpropionate, salicylate, succinate, sulfate, bisulfate, pyrosulfate, sulfite, bisulfite, sulfonate, benzenesulfonate, p-bromophenylsulfonate, chlorobenzene sulfonate, ethanesulfonate, 2-hydroxyethanesulfonate, methanesulfonate, naphthalene-1-sulfonate, naphthalene-2-sulfonate, p-toluenesulfonate, xylenesulfonate, tartrate, or the like. Bisulfate salts are preferred.

The method for preparing (S)-Clopidogrel, or derivatives of salts thereof according to the present invention includes a simple process based on hydrolysis of 2-chlorophenylglycine alkyl ester, and allows easy recovery of the desired product after the reaction. Further, the method according to the present invention is highly cost-efficient, since the (S)-2-chlorophenylglycine or (S)-chlorophenylglycine alkyl ester intermediate obtained from the hydrolysis of 2-chlorophenylglycine alkyl ester and having high optical purity is used to provide (S)-Clopidogrel and derivatives thereof having high optical purity.

### Example

The examples will nowbe described. The following examples are for illustrative purposes only and not intended to limit the scope of the present invention.

### Example 1: Preparation of Racemic 2-Chlorophenyl Glycine Alkyl Ester

To 500 ml of methanol, 100 g of racemic 2-chlorophenylglycine is introduced and 70 ml of thionyl chloride is added gradually dropwise thereto at -10°C. Next, the reaction mixture is warmed to 50°C and agitated for 4 hours. After the agitation, the reaction mixture is concentrated under reduced pressure to remove volatile materials. Then, 1L of methylene chloride and 100 ml of water are added to dilute the mixture, which, in turn, is neutralized with aqueous NaOH. The resultant organic layer is washed with 500 ml of aqueous saturated NaHCO₃ solution. From the washed mixture, methylene chloride layer is separated, dried over MgSO₄ and concentrated under reduced pressure to obtain 104.6 g of racemic 2-chlorophenylglycine methyl ester 1a (yield: 97.3%).

Meanwhile, butanol is used instead of methanol and the reaction is carried out under the same condition as described above to obtain 120.4 g of racemic 2-chlorophenylglycine butyl ester 1b (yield: 95.2%). Compounds 1a and 1b are analyzed by NMR spectrometry and the results are as shown below.

1a : ¹H-NMR (CDCl₃, 300MHz) = 7.4-7.2 (m, 4H), 5. 00 (s, 1H), 3.72(s, 3H), 1.95(s, 2H).

1b : ¹H-NMR (CDCl₃, 300MHz) = 7.4-7.2 (m, 4H), 5. 00 (s, 1H), 4.13(t, 2H), 1.95(s, 2H), 1.59-1.50(m, 2H), 1.31-1.19(m, 2H), 0.84(t, 3H).

### Example 2: Preparation of Optically Active (S)-2-chlorophenylglycine Alkyl Ester via Hydrolysis

To a reactor containing 1,000 ml of 0.1M potassium phosphate buffer (pH 7.5), 200 g of racemic 2-chlorophenylglycine methyl ester 1a obtained from Example 1 is introduced, and 10 ml (1%, v/v) of alcalase 2.5L enzyme is added thereto. The reaction mixture is allowed to react at 30°C under 250 rpm. To maintain a constant pH, 2N aqueous NaOH solution is introduced to the reaction mixture. After carrying out the reaction for 24 hours, 1,000 ml of ethyl acetate is added to the reaction mixture to extract optically active (S)-2-chlorophenylglycine methyl ester 2a having an optical purity of at least 99%, which, in turn, is dried over MgSO₄ and concentrated under reduced pressure to obtain 85.6 g of (S)-2-chlorophenylglycine methyl ester 2a having an optical purity of 99.8% ee (yield: 85.6% Vs. (S)-enantiomer).

Meanwhile, racemic 2-chlorophenylglycine butyl ester 1b is used instead of racemic 2-chlorophenylglcine methyl ester 1a, and the reaction is carried out under the same condition as described above to obtain 57.2 g of (S)-2-chlorophenylglycine butyl ester 2b having an optical purity of 99.2% ee (yield: 57.2% Vs. (S)-enantiomer). The optical purity of each compound is analyzed by using liquid chromatography (Model 1525, Waters) equipped with Chirosil RCA(+) chiral column (150X4.6 mm, 5 µm) in the form of optically active crown ether. The compounds are analyzed by NMR spectrometry and the results are as shown below.

1a : ¹H-NMR (CDCl₃, 300MHz) = 7.4-7.2 (m, 4H), 5.00(s, 1H) , 3.72(s, 3H), 1.95(s, 2H).

1b : ¹H-NMR (CDCl₃, 300MHz) = 7.4-7.2 (m, 4H), 5. 00 (s, 1H), 4. 13 (t, 2H), 1.95(s, 2H), 1 .59-1 .50 (m, 2H), 1 .31-1 . 19 (m, 2H), 0.84 (t, 3H).

### Example 3: Preparation of Optically Active (S)-Alkyl -α-(2-thienylethylamino)(2-chlorophenyl)acetate hydrochloride

First, 100 g of (S)-2-chlorophenylglycine methyl ester 2a obtained from Example 2 is dissolved into 1,000 ml of acetonitrile, 60 g of potassium bicarbonate and 156 g of paratoluene sulfonate are added thereto, and the resultant mixture is refluxed for 20 hours. The mixture is depressurized to remove volatile materials therefrom and dissolved into ethyl acetate, and then the organic layer is washed with distilled water. After the separation of the organic layer, concentrated hydrochloric acid is added at 0°C to perform precipitation of crystals, which, in turn, is dried under vacuum to provide 125.4 g of (S)-methyl-α-(2-thienylethylamino)(2-chlorophenyl) acetate 3a in the form of hydrochloride (yield: 72.3%).

Meanwhile, (S)-2-chlorophenylglycine butyl ester 2b is used instead of (S)-2-chlorophenylglycine methyl ester 2a, and the reaction is carried out under the same condition as described above to provide 88.1 g of (S)-butyl- α-(2-thienyl ethylamino)(2-chlorophenyl) acetate 3b in the form of hydrochloride (yield: 54.8%). The compounds are analyzed by NMR spectrometry and the results are as shown below.

3a : ¹H-NMR (CDCl₃, 300MHz) = 7 .40-7.32 (m, 2H), 7.27-7 .21 (m, 2H), 7 .12 (d, 1H), 6. 92 (dd, 1H), 6.82(d, 1H), 4.93(s, 1H), 3.69(s, 3H), 3. 03 (t, 2H), 2.96-2.88(m, 1H), 2.82-2.74(m, 1H), 2.18(s, 1H).

3b :¹H-NMR(CDCl₃, 300MHz) =7.37-7.33 (m, 2H), 7.27-7.21(m, 2H), 7.12(d, 1H), 6.91(dd, 1H), 6.82(d, 1H), 4.91(s, 1H), 4.09(t, 2H), 3.03(t, 2H), 2.96-2.88(m, 1H), 2.83-2.74(m, 1H), 2.35(s, 1H), 1.57-1.48(m, 2H), 1.29-1.19(m, 2H), 0.83(t, 3H).

### Example 4: Preparation of Optically Active (S)-Clopidogrel Alkyl Derivative Bisulfate

To 200 g of (S) -methyl-a - (2-thienylethylamino) (2-chloro phenyl)acetate 3a obtained from Example 3, 600 ml of 35% aqueous formaldehyde solution is added and the mixture is refluxed for 4 hours. Next, the reaction mixture is diluted with ethyl acetate and washed with aqueous saturated NaHCO₃ solution, and then the organic layer is separated from the reaction mixture. The organic layer is concentrated under reduced pressure to remove the solvent and dissolved into acetone. Then, concentrated sulfuric acid is added thereto at 0°C to perform precipitation of crystals. The precipitated crystals are filtered off, followed by vacuum drying, thereby providing 179.7 g (yield: 83.9%) of (S)-Clopidogrel 4a in the form of bisulfate with an optical purity of 99.5% ee.

Meanwhile, (S)-butyl-α-(2-thienylethylamino)(2-chloro phenyl)acetate 3b hydrochloride is used instead of (S)-methyl-α- (2-thienylethylamino)(2-chlorophenyl)acetate 3a hydrochloride, and the reaction is carried out under the same condition as described above, thereby providing 143.4 g (yield: 67.4%) of (S)-Clopidogrel butyl ester derivative 4b having an optical purity of 99.0% ee. The optical purity of each compound is analyzed by using liquid chromatography (Model 1525, Waters) equipped with Ultron ES-OVM column (150x4.6 mm, 5 µm, Ovomucoid). The compounds are analyzed by NMR spectrometry and the results are as shown below.

4a : ¹H-NMR (CDCl₃, 300MHz) = 7.72 (dd, 1H), 7.41 (dd, 1H), 7.33-7.23 (m, 2H), 7.06(d, 1H), 6.67(d, 1H), 4.94 (s, 1H), 3.73 (s, 3H), 3.80-3.62(dd, 2H), 2.89(s, 4H).

4b : ¹H-NMR (CDCl₃, 300MHz) = 7.72(dd, 1H) , 7.40(dd, 1H) , 7.29-7.24(m, 2H), 7.06(d, 1H), 6.67(d, 1H), 4.89(s, 1H), 4.13(t, 2H), 3.78-3.60(dd, 2H), 2.89(s, 4H), 1.60-1.50(m, 2H), 1.32-1.19(m, 2H), 0.86(t, 3H).

### [Industrial Applicability]

As described above, the method for preparing (S)-Clopidogrel and derivatives thereof according to the present invention includes simple processing steps, and uses only a small amount of enzyme with no need for an optical isolating agent, and thus shows high cost efficiency. In addition, the method according to the present invention utilizes (S)-2-chlorophenylglycine alkyl ester having high optical purity as an intermediate, and thus is amenable to mass production of Clopidogrel and derivatives thereof having high optical purity. Further, the method uses no toxic materials, and thus shows high eco-friendly characteristics.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the present invention as set forth in the appended claims.

## Claims

1. A method for preparing (S)-Clopidogrel represented by Chemical Formula 1, or derivatives or salts thereof, comprising the steps of:
(a) subjecting a racemic 2-chlorophenylglycine alkyl ester compound represented by Chemical Formula 9 to enzymatic hydrolysis to obtain an optically active compound represented by the following Chemical Formula 10;
(b) reacting the optically active compound represented by Chemical Formula 10 with a compound represented by the following Chemical Formula 11 to obtain a compound represented by the following Chemical Formula 12; and
(c) carrying out cyclization of the compound represented by Chemical Formula 12 with a formylating agent in the presence of an acid: 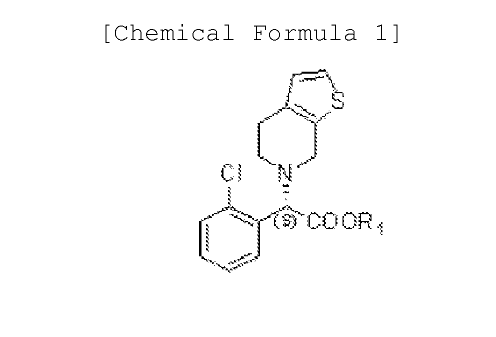 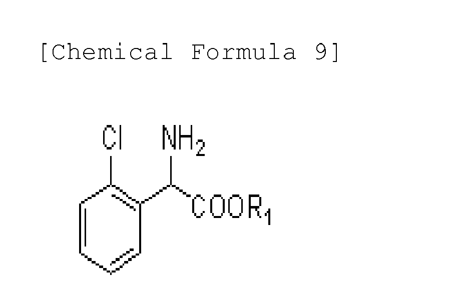 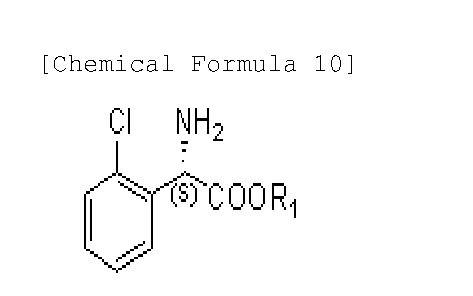 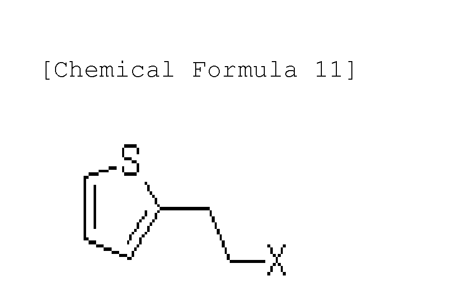 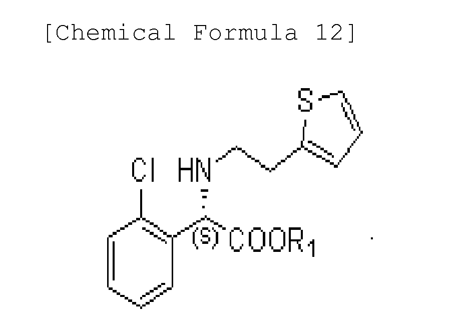
wherein R₁ is H, substituted or non-substituted C1-C8 alkyl, substituted or non-substituted C1-C8 alkenyl, benzyl or C3-C6 cycloalkyl; and X is a halogen atom selected from the group consisting of fluorine (F), chlorine (Cl), bromine (Br) and iodine (I) or -OSO₂R₂ (wherein R₂ is substituted or non-substituted C1-C8 alkyl, substituted or non-substituted aryl, substituted or non-substituted arylalkyl, substituted or non-substituted heteroaryl or substituted or non-substituted nheteroarylalkyl).

2. The method of claim 1, wherein the enzyme is hydrolase.

3. The method of claim 1, wherein the formylating agent is selected from the group consisting of formaldehyde, formaldehyde hydrate and formaldehyde polymers.

4. The method of claim 1, wherein the acid in step (c) is selected from the group consisting of organic acids, inorganic acids and mixtures thereof.

5. The method of claim 1, wherein the hydrolysis is carried out under pH 4-10 at a temperature of 10-70°C.

6. The method of claim 1, wherein step (a) further includes separating (R)-2-chlorophenylglycine and (R)-2-chlorophenyl glycine alkyl ester produced after the hydrolysis by using a solvent.

7. The method of claim 6, wherein the solvent is selected from the group consisting of ethyl acetate, methylene chloride, diethyl ether, diisopropyl ether, tert-butyl methyl ether, 1,2-dimethoxyethane, 1,2-dichloroethane, benzene, toluene, xylene and mixtures thereof.
